# EUROPEAN PATENT APPLICATION

(11) **EP 3 967 292 A1**
(43) Date of publication of application: **16.03.2022**
(21) Application number: 20195947.5
(22) Date of filing: 14.09.2020
(51) Int. Cl.: A61K 8/25, A61Q 17/04, A61K 8/9706

(54) **BENTHIC PENNATE DIATOM FRUSTULES AS AN SPF BOOSTER**

(71) Applicant: Swedish Algae Factory AB, 412 92 Göteborg (SE)
(72) Inventor: Allert, Sofie, 423 63 Torslanda (SE); Andrén, Lizette, 413 04 Göteborg (SE); Rova, Emma, 416 49 Göteborg (SE)
(74) Representative: Henricson Briggs, David James

(57) **Abstract**

A cosmetic composition comprising: at least one sunscreen active agent; and, benthic pennate diatom frustules. Benthic pennate diatom frustules for use as an SPF booster.

## Description

### Field of the Invention

The present disclosure relates to compositions comprising benthic pennate diatom frustules as an SPF Booster. In particular it relates to compositions comprising a sunscreen active agent and benthic pennate diatom frustules.

### Background of the invention

Sunscreens are used as cosmetic products to protect the skin from harm caused by UV radiation. A common measure for determining the protective value of a sunscreen is the Sun Protection Factor (SPF) of the sunscreen. Sun Protection Factor (SPF) is defined as the ratio of the amount of energy required to produce a minimal erythema on sunscreen protected skin to the amount of energy required to produce the same level of erythema on unprotected skin.

The UV radiation causing damage to the skin may be both UVA and UVB rays.

Cosmetic products having an increased or boosted the SPF are important to provide better protection to the skin from the harmful UV radiation.

### Summary of the invention

Accordingly, the present invention preferably seeks to mitigate, alleviate or eliminate one or more of the above-identified deficiencies in the art and disadvantages singly or in any combination and solves at least the above mentioned problems by providing a cosmetic composition comprising: at least one sunscreen active agent; and, benthic pennate diatom frustules.

The use of benthic pennate diatom frustules as an SPF booster is also provided.

A method of preparing a sunscreen comprising benthic pennate diatom frustules is also provided.

Further advantageous embodiments are disclosed in the appended and dependent patent claims.

### Brief description of the figures

The figures show results from the experiments described in the experimental section.
Fig. 1 is the MPF values obtained for a Reference SPF composition. The figure shows 9 scans and an average MPF for a typical plate of the 5 scanned plates.
Fig. 2 is the MPF values obtained for a control composition comprising no benthic pennate diatoms. The figure shows 9 scans and an average MPF for a typical plate of the 5 scanned plates.
Fig. 3 is the MPF values obtained for a composition comprising 0.1% benthic pennate diatoms. The figure shows 9 scans and an average MPF for a typical plate of the 5 scanned plates.
Fig. 4 is the MPF values obtained for a composition comprising 0.3% benthic pennate diatoms. The figure shows 9 scans and an average MPF for a typical plate of the 5 scanned plates.

### Detailed description

The present disclosure relates to a cosmetic composition comprising at least one sunscreen active agent and benthic pennate diatom frustules. The benthic pennate diatom frustules boost the protective value of the sunscreen (SPF) compared to compositions comprising no benthic pennate diatom frustules. The benthic pennate diatom frustules are thereby an SPF booster.

Diatoms are a type of algae which are present in both fresh water and marine environments. The diatoms comprise a frustule which has a silicon dioxide content. The frustules are a source of nanoporous silicon dioxide. Benthic diatoms are diatoms which grow attached to surfaces at the water-sediment interface, this is as opposed to pelagic diatoms which grow in open water. Pennate diatoms are diatoms which are bilaterally symmetrical as opposed to radially symmetrical diatoms, which are defined as centric diatoms.

The light manipulating properties of benthic pennate diatom frustules have been previously noted in solar cell applications where they enhance the efficiency of solar cells. See for example, WO 2017/211892 A1 (Swedish Algae Factory AB). However, there has been to-date no suggestion that benthic pennate diatoms may be used in cosmetic compositions as an SPF booster.

Cosmetic compositions comprising fossilised frustules, that is diatomaceous earth, are known in the field. The diatomaceous earth is used in such processes as bulking, opacifying or anti-caking agent. In use as, for example, and opacifying agent the diatomaceous earth may have a UV absorbing effect. As opposed to fossilised diatom frustules, i.e., diatomaceous earth, benthic pennate diatom frustules have an SPF boosting effect which is significantly greater than that which could be expected for a similar amount of diatomaceous earth. This is evidenced by the experimental section below, where a cosmetic composition comprising only 0.1% benthic pennate diatoms had an almost 22% greater SPF than the control composition comprising no benthic pennate diatoms.

Without wishing to be bound by theory the inventors contend that the specific structure and form of the benthic pennate diatom frustules has both a UV absorbing, UV refracting, and UV conversion effect. The UV conversion effect may be the conversion of UV light from UV wavelengths to light in the visible spectrum (e.g., blue) via silica photoluminescence. Such SPF boosting activity has not been shown in fossilised diatom frustules. Centric pelagic diatoms frustules of *C. wailesii* have been shown to emit blue light when excited with light at a wavelength of 325 nm (De Tommasi, E. et al. UV-shielding and wavelength conversion by centric diatom nanopatterned frustules. Sci Rep 8, 16285 (2018)). As benthic pennate diatoms are less exposed to UV radiation in the natural environment it is generally considered that they are less likely to exhibit UV protective capabilities and rather evolved to migrate downwards, away from UV radiation. (De Tommasi, E. et al. Sci Rep 8, 16285 (2018)). The present results indicate that benthic pennate diatom frustules have an SPF boosting activity which was not expected from benthic pennate diatom frustules. It is thus contended that the UV conversion process may be occurring with the benthic pennate diatoms in the present composition.

The composition may comprise at least 0.05% by weight benthic pennate diatom frustules. The composition may comprise from 0.1% to about 3% by weight benthic pennate diatom frustules. The composition may comprise from about 0.1% to about 1% by weight benthic pennate diatom frustules. The composition may comprise from about 0.1% to about 0.5% by weight benthic pennate diatom frustules, such as from about 0.1% to about 0.3%. The benthic pennate diatom frustules are extracted from diatoms and are not fossil diatom frustules.

The composition may comprise benthic pennate diatom frustules at an amount which forms a monolayer when applied to a surface.

The frustules may be extracted from cultured and harvested benthic pennate diatoms in accordance with the process described in WO 2017/211892 A1 (Swedish Algae Factory AB). The frustules may be extracted via a chemical extraction process whereby the organic biomass of the diatom is removed via a chemical agent leaving the frustules.

The present composition comprises at least one sunscreen active agent. The at least one sunscreen active agent may be an inorganic sunscreen active agent or an organic sunscreen active agent, or a combination thereof. The composition may comprise a plurality of different inorganic or organic sunscreen active agents. The compositions PE1 to PE3 of the experimental section comprise a plurality of physical blocking inorganic sunscreen active agents. The combination of different active agents may provide improved sun protective power over a range of UV wavelengths.

Inorganic sunscreen active agents may be for example, zinc oxide, titanium dioxide, iron oxide, zirconium oxide, cerium oxide. Inorganic sunscreen active agents may be nanopigments formed from coated metal oxides as those disclosed herein. Preferably the inorganic sunscreen active agent is zinc oxide, titanium dioxide or a mixture thereof. The inorganic sunscreen active may be a transparent inorganic metal oxide, such as transparent zinc oxide or transparent titanium dioxide. As is known in the art, transparent inorganic metal oxides are metal oxides which have been prepared in such a manner that they appear transparent to visible light and therefore do not appear white when applied to the skin. For the sake of clarification, the inorganic sunscreen agent may be a transparent metal oxide or a non-transparent metal oxide.

The sunscreen active agent may be an organic sunscreen active agent, such as anthranilates, cinnamic derivatives, dibenzoylmethane derivatives, salicylic derivatives, camphor derivatives, triazine derivatives, benzophenone derivatives, β,β-diphenylacrylate derivatives, benzotriazole derivatives, benzalmalonate derivatives, benzimidazole derivatives, imidazolines, bisbenzoazolyl derivatives, p-aminobenzoic acid (PABA) derivatives, methylenebis(hydroxyphenylbenzotriazole) derivatives, screening polymers and screening silicones, dimers derived from α-alkylstyrene, 4,4-diarylbutadienes; and their mixtures.

Benthic pennate diatom frustules have the advantage over other known SPF boosters such as polymer-based SPF boosters in that they are natural, i.e., they are naturally occurring. Cosmetic products with natural ingredients are often preferred by users as they are considered less harmful to the body, and/or to the environment. The benthic pennate diatoms may be extracted from cultured diatoms grown according to the process described in WO 2017/211892 A1 (Swedish Algae Factory AB). The benthic pennate diatoms may be cultured in, water comprising at least a portion of waste-water from for example, fish farms or food processing plants. Such waste-water has an increased nitrogen content which may be ideal for culturing the diatoms. Such a manufacturing process provides an SPF booster which is both an effective SPF booster and a means to recycle waste-water. To this end, the benthic pennate diatom frustules are especially suitable as SPF boosters in sunscreen compositions comprising inorganic, UV blocking sunscreen active agents, as such sunscreen compositions are often marketed as natural sunscreens or chemical free sunscreens. As would be understood, "chemical free" in this context refers to the lack of chemical absorbing agents such as organic sunscreen active agents, for example p-aminobenzoic acid (PABA) derivatives.

The sunscreen active agents absorb radiation in the UVA and/or UVB spectrum. The UVA spectrum is the 280 nm to 320 nm region of the ultraviolet light spectrum. The UVB spectrum is the 320 nm to 400 nm region of the ultraviolet light spectrum.

The at least one sunscreen active agent is generally present in an amount from about 0.1% to about 75% by weight of the composition. The sunscreen active agent may be present in an amount from about 1% to about 40% by weight of the composition, such as about 5% to about 25%. The sunscreen active agent may be present in an amount from about 10% to about 15% by weight of the composition. Due to the presence of the benthic pennate diatom frustules in the composition, the sunscreen active agent may be present in an amount less than generally needed to achieve a specific SPF in a composition.

The compositions may also comprise water and/or oil. The compositions may be an emulsion comprising water. The compositions may be in the form of a water-in-oil emulsion or an oil-in-water emulsion. Water or oil may be present in amounts from about 5% to about 70% by weight of the composition.

The composition may be a powdered sunscreen composition comprising benthic pennate diatom frustules. A powdered or powder sunscreen composition is a composition comprising fine non-agglomerated particles in a carrier medium. Powdered sunscreen compositions are known in the art. Benthic pennate diatom frustules are especially suitable for use in powdered sunscreen compositions as the frustules are a dry powder.

The composition may comprise other ingredients such as emollients, humectants, lubricating agents, conditioning agents, surfactants, plasticizers, preservatives, fillers, active ingredients that are not sunscreen active agents, perfumes, thickeners, antioxidants, vitamins and other ingredients which are known and commonly used in cosmetic compositions. Compositions may comprise metal oxides in addition to the inorganic sunscreen active agents listed above. The compositions may comprise for example, alumina, mica. Compositions comprising benthic pennate diatoms may also comprise silica, separate and in addition to the silicon oxide frustules, for cosmetic application purposes.

### Experimental Section

The boosting efficacy of benthic pennate diatoms to a UV filter composition was determined via *in vitro* studies. The Sun Protection Factor (SPF) of a known composition was compared to the SPF of compositions comprising benthic pennate diatom frustules at various concentrations.

### General description

The test products were spread onto a thin film on a suitable synthetic substrate and the UV absorbance through this film is measured with a spectrophotometer. The sun protection factor was determined by firstly irradiating the product with 4 Minimum Erythemal Doses (MEDs) followed by the scanning of the sample from 290nm to 400nm. During the scanning the obtained data was accumulated and stored at intervals of 1nm to determine the monochromatic protection factor (MPF) for each of the selected wavelengths. Then the MPF is used to calculate the SPF value, using solar irradiance and erythemal constants.

### Sample Preparation

Benthic pennate diatom frustules were added to an existing sunscreen formulation, whereby the water content by weight was adjusted to compensate for the addition of the benthic pennate diatom frustules. The existing sunscreen formulation comprised an inorganic sunscreen active agent.

The following compositions were prepared.

**Table 1: The different compositions PE1-PE3 and the Reference SPF composition**

| Composition | Benthic pennate diatom frustule (% wt.) | Purified Water (% wt.) | Inorganic sunscreen active agent (Zinc oxide, Titanium dioxide, Silica) (% wt.) | Other (% wt.) |
|---|---|---|---|---|
| PE1 (Control, 0%) | 0% | 44.40% | 12% | 43.60% |
| PE2 (0.1%) | 0.1% | 44.30% | 12% | 43.60% |
| PE3 (0.3%) | 0.3% | 44.10% | 12% | 43.60% |
| Reference SPF composition (SPF *in vivo* 16.1 +/- 2.4) | | | | |

**Table 2: Other ingredients of the compositions PE1 to PE3**

| | Amount (% wt.) |
|---|---|
| Preservative (glycerine, aqua, sodium levulinate, sodium anisate) | 4% |
| Glycerine | 2% |
| Magnesium sulphate | 1% |
| Lactic acid | 0.1% |
| Squalane | 8% |
| Seed oils | 12% |
| Silk | 8% |
| Emulsion (water in oil) | 8% |
| Vitamin E | 0.5% |

Moulded PMMA plates, 50mmx50mm, (ref.: HEL-PMMA55 (HelioScience, France) were used as a substrate. The foil covered PMMA plates were uncovered and cleaned with an antistatic cloth. The PMMA plates were weighed. The above compositions (PE1-PE3, SPF reference) comprising benthic pennate diatom frustules at various concentrations were applied to the PMMA plate. Five plates of each of PE1-PE3 were prepared. Four plates of Reference SPF composition were prepared. Approximately 1.3 mg/cm2 of each composition was applied to the surface of the PMMA plate. Total mass of composition on each plate was 32.5 ± 0.3 mg. The compositions were spread over the surface of the plate with a saturated gloved finger. The plates with the compositions applied to the surface were allowed to dry for at least 30 mins in the dark at the same temperature, 30.0±5.0°C and relative humidity of 50.0±10.0%. The same temperature and humidity conditions were used for the UV exposure and absorbance measurements.

### Equipment

The Irradiation source was a Solar Simulator PV Cell testing with model number 16S-300-002 (Solarlight^{®}). The irradiation source was calibrated according to the manufacturer's specifications.

UV spectrophotometer, SPF-290AS (Solarlight^{®}) was used to perform all absorbance measurements. The UV spectrophotometer was calibrated according to the manufacturer's specifications.

UVB radiometer (SUV Detector PMA2101S-UVS, Solarlight^{®}) was calibrated against a spectroradiometric measurement of the solar simulator output.

Dose controller/meter (DCS 2.0 Dose Controller/Meter, Solarlight^{®}) was calibrated according to the manufacturer's specifications.

### UV test of known SPF composition

Prior to each test of PE1-PE3, each of four PMMA plates treated with the Reference SPF composition were tested to ensure the equipment was maintained within expected limits. The PMMA plate with the reference composition was placed 45.7 cm from the UV beam. The time period to irradiate the PMMA plate with 4 MEDs was determined via the dose controller. The SPF reference PMMA plate was placed in the UV spectrophotometer and the mean MPF values were recorded with 9 measurements at 9 different locations on the PMMA plate for each wavelength over the full UV spectrum (290 to 400 nm). The SPF in vitro value was compared to the in vivo known reference to ensure values are within the expected range. The upper and lower limits were established for the reference composition according to ISO 24444. The measured in vitro SPF value for the Reference SPF composition must be within the range 13.7 to 18.5.

### UV test of benthic pennate diatom compositions.

Each of the five PMMA plates for each of PE1-PE3 were placed 45.7 cm from the UV beam. Five plates of each of PE1-PE3 were placed in the UV spectrophotometer. The mean MPF values were recorded via 9 measurements at 9 different locations on each of the PMMA plates for each wavelength over the full UV spectrum (290 to 400 nm). The MPF values were recorded at 1 nm intervals. The mean values for SPF are shown below in the results section.

### Results

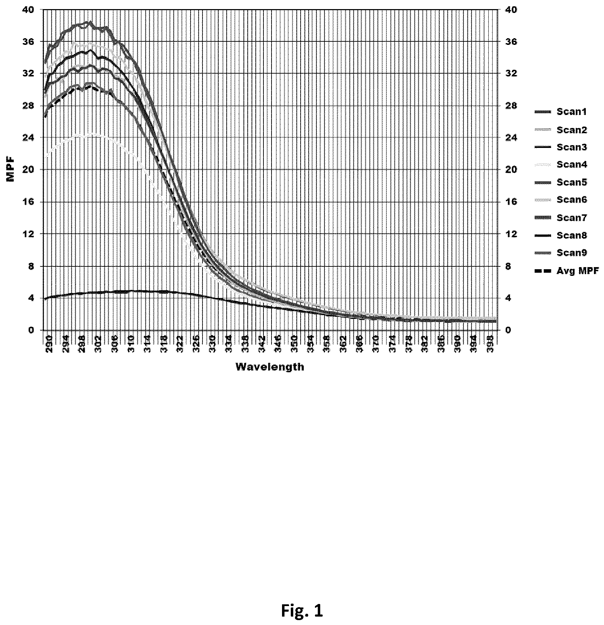

The MPF values obtained for the Reference SPF composition for an example plate are shown in Figure 1.

**Table 3: Mean SPF for Reference SPF composition**

| PMMA Plate no | Mean SPF (9 scans) |
|---|---|
| 1 | 13.18 |
| 2 | 12.88 |
| 3 | 12.25 |
| 4 | 14.56 |
| 5 | 16.99 |
| Mean SPF ± SD | 13.97 ± 1.89 |

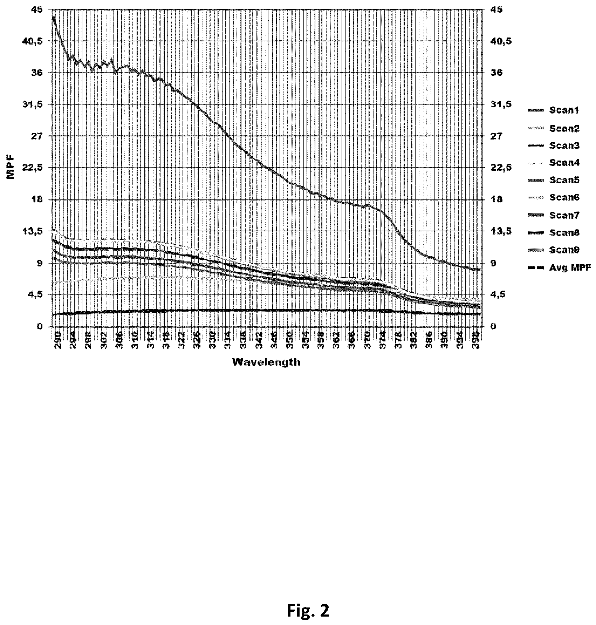

The MPF values obtained for PE1 for an example plate are shown in Figure 2.

**Table 4: Mean SPF for PE1 (0% benthic pennate diatom frustules)**

| PMMA Plate no | Mean SPF (9 scans) |
|---|---|
| 1 | 13.15 |
| 2 | 10.68 |
| 3 | 16.10 |
| 4 | 17.18 |
| 5 | 11.24 |
| Mean SPF ± SD | 13.67 ± 2.89 |

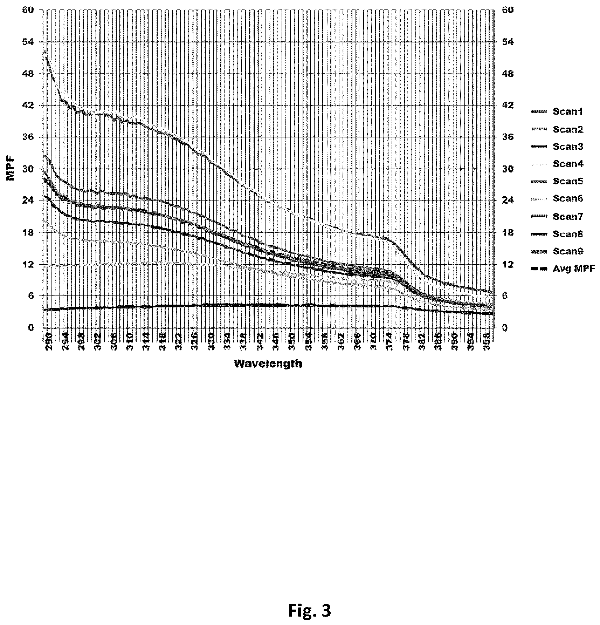

The MPF values obtained for PE2 for an example plate are shown in Figure 3.

**Table 5: Mean SPF for PE2 (0.1% benthic pennate diatom frustules)**

| PMMA Plate no | Mean SPF (9 scans) |
|---|---|
| 1 | 14.54 |
| 2 | 16.99 |
| 3 | 20.30 |
| 4 | 14.40 |
| 5 | 17.59 |
| Mean SPF ± SD | 16.76 ± 2.44 |

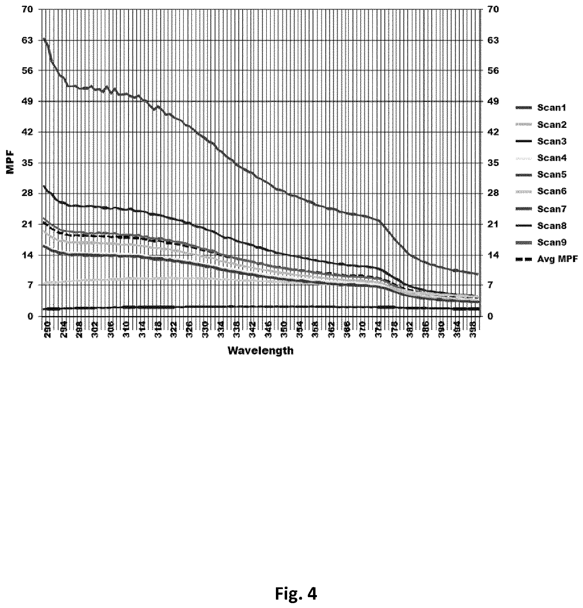

The MPF values obtained for PE3 for an example plate are shown in Figure 4.

**Table 6: Mean SPF for PE3 (0.3% benthic pennate diatom frustules)**

| PMMA Plate no | Mean SPF (9 scans) |
|---|---|
| 1 | 17.39 |
| 2 | 16.52 |
| 3 | 13.06 |
| 4 | 14.69 |
| 5 | 14.90 |
| Mean SPF ± SD | 15.31 ± 1.69 |

**Table 8: Mean SPFfor each composition**

| PMMA plate | Mean SPF | Standard Deviation | SPF increase (%) with respect to PE1 |
|---|---|---|---|
| SPF reference | 13.97 | 1.89 | N/A |
| PE1 (0%) | 13.67 | 2.89 | N/A |
| PE2 (0.1%) | 16.76 | 2.44 | 21,99% |
| PE3 (0.3%) | 15.31 | 1.69 | 11,99% |

### Discussion

As can be seen from the above results, compositions comprising benthic pennate diatom frustules have an SPF effect. Compositions comprising benthic pennate diatom frustules in concentrations of 0.1% (PE2) and 0.3% (PE3) have significantly higher mean SPF than the control composition (PE1).

As can be seen from the above table, the standard deviation decreased successively with each increase in the amount of benthic pennate diatoms. This indicates that benthic pennate diatom frustules may have a more consistent boosting effect at increased ratios.

Furthermore, it should be noted that the sunscreen active agent in the specific test was a mineral was present in an amount of 12% in the compositions PE1-PE3. The addition of only 0.1% benthic pennate diatoms provided therefore a remarkable benefit.

Without wishing to be bound by theory, an amount of benthic pennate diatom frustules which can form a monolayer of frustules when applied as a composition may be the optimal amount with respect to SPF boosting effect. This implies that the boosting effect of benthic pennate diatom frustules may not necessarily be greater at significantly increased amounts in the composition. The optimal amount which has the greatest SPF boosting effect, which may be the amount which forms a monolayer when applied to a surface, may depend on the specific surface and the specific composition.

Although, the present invention may have been described above with reference to specific or formulations, it is not intended to be limited to the specific form set forth herein. Rather, the invention is limited only by the accompanying claims.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality.

## Claims

1. A cosmetic composition comprising:
- at least one sunscreen active agent; and,
- benthic pennate diatom frustules.

2. The cosmetic composition according to claim 1, wherein the composition comprises at least 0.05% benthic pennate diatom frustules.

3. The cosmetic composition according to claims 1 or 2, wherein the composition comprises from about 0.05% to about 1% benthic pennate diatom frustules.

4. The cosmetic composition according to any of claims 1 to 3, wherein the sunscreen active agent is present in an amount of from 0.1% to 75%, such as from 1% to 50%.

5. The cosmetic composition according to any of claims 1 to 4, wherein the sunscreen active agent is at least one inorganic sunscreen active agent, selected from the list comprising: titanium dioxide, zinc oxide, iron oxide, zirconium oxide, cerium oxide; or, a combination thereof.

6. The cosmetic composition according to any of claims 1 to 5, wherein the sunscreen active agent is at least one organic sunscreen active agent.

7. The cosmetic composition according to any of claims 1 to 6, wherein the cosmetic composition is provided in acceptable cosmetic carrier medium.

8. The cosmetic composition according to any of claims 1 to 7, wherein the acceptable cosmetic carrier medium is water or oil.

9. The cosmetic composition according to any of claims 1 to 8, wherein the cosmetic composition is provided in powdered form.

10. Benthic pennate diatom frustules for use as an SPF booster in a sunscreen.

11. The composition according to claims 1 to 9, or the benthic pennate diatom frustules for use according to claim 10, wherein the frustules are extracted from cultured and harvested benthic pennate diatoms.

12. A method of boosting the SPF of a sunscreen composition comprising:
- including benthic pennate diatom frustules.

13. The method according to claim 12, wherein the benthic pennate diatom frustules are included in an amount from about 0.05% to about 1% by weight of the composition.

14. The method according to claim 12 or 13, wherein the sunscreen has an SPF that is at least 10% higher than a corresponding sunscreen in which benthic pennate diatoms frustules are not present.
